(19) Europäisches Patentamt
European Patent Office
Office européen des brevets

(11) Publication number: 0 503 203 A1

# (12) EUROPEAN PATENT APPLICATION

(21) Application number: 91400713.3

(51) Int. Cl.[5]: C07K 5/08, A61K 37/64

(22) Date of filing: 15.03.91

(43) Date of publication of application:
16.09.92 Bulletin 92/38

(84) Designated Contracting States:
FR

(71) Applicant: MERRELL DOW PHARMACEUTICALS INC.
P.O. Box 156300 2110 East Galbraith Road
Cincinnati Ohio 45215-6300(US)

(72) Inventor: Neises, Bernhard
Flosserweg 5
W-7600 Offenburg-Griesheim(DE)
Inventor: Ganzhorn, Axel
2, Rue René Descarters
F-67380 Lingolsheim(FR)

(74) Representative: Gillard, Marie-Louise et al
Cabinet Beau de Loménie 55, Rue d'Amsterdam
F-75008 Paris(FR)

## (54) Novel thrombin inhibitors.

(57) This invention relates to novel polyfluorinated alkyl derivatives of certain tripeptides, to the methods for their preparation, the intermediates therefore, to their use in inhibiting thrombin and lung tryptase and in their end-use application as anti-coagulants useful in treating thrombophlebitis and coronary thrombosis and in the treatment of asthma and human immunodeficiency virus.

EP 0 503 203 A1

This invention relates to novel polyfluorinated alkyl derivatives of certain tripeptides, to the methods for their preparation, the intermediates therefore, to their use in inhibiting thrombin and lung tryptase and in their end-use application as anti-coagulants useful in treating thrombophlebitis and coronary thrombosis and in the treatment of asthma and human immunodeficiency virus.

More specifically this invention relates to the novel compounds of the formula

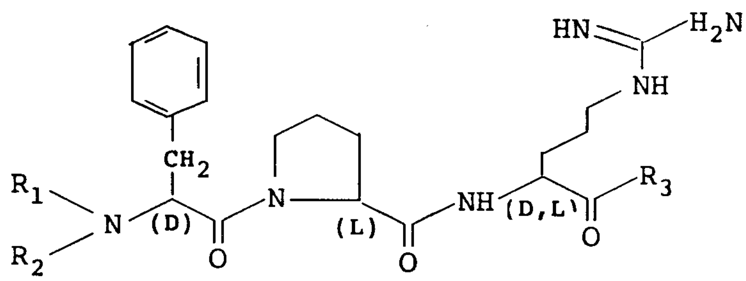

I

the hydrates thereof, and the pharmaceutically acceptable salts thereof wherein

$R_1$ is H or $C_{1-7}$ alkyl,

$R_2$ is H or $C_{1-7}$ alkyl, or $R_1$ and $R_2$ taken together with the nitrogen atom to which they are attached form a 5- or 6-membered heterocycle,

$R_3$ is $CF_3$ or $CF_2CF_3$, with the proviso that when $R_1$ and $R_2$ are both H, then $R_3$ is other than $CF_3$ or $CF_2CF_3$.

The natural amino acids, with the exception of glycine, contain a chiral carbon atom. Unless otherwise specifically indicated, the optically active amino acids, referred to herein, are of the L-configuration. As indicated, it is preferred that Phe be in its D-configuration.

The tripeptides of formula 1 can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, trifluoroacetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid.

An alkyl group is taken to include straight, branched, or cyclic alkyl groups, for example, methyl, ethyl, propyl, isopropyl, butyl, isobutyl, tert-butyl, pentyl, isopentyl, sec-pentyl, cyclopentyl, hexyl, isohexyl, cyclohexyl and cyclopentylmethyl. In the instance wherein $R_1$ and $R_2$ are lower alkyl, it is preferred that one or both be methyl. When $R_1$ and $R_2$ form a 5-6-membered heterocycle with the nitrogen atom to which they are attached the preferred moieties are pyrrolidine and piperidine. Preferred compounds are those wherein at least one of $R_1$ or $R_2$ is methyl.

It is also convenient to express the definition of the compounds of Formula I with the following formulae

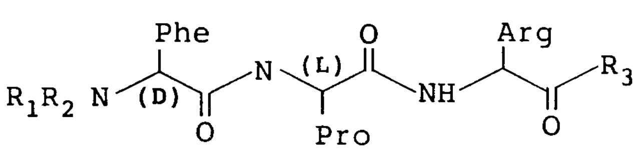

**2A**

and

$R_1R_2$Phe-ProArg$R_3$ 2B

wherein $R_1$, $R_2$ and $R_3$ are as previously defined. In 2A the Phe, Pro and Arg represent the residues of the α-amino acids of phenylalanine, proline and arginine, and in 2B, the Phe, Pro and Arg are the α-amino acids of phenylalanine, proline and arginine with the $R_1R_2$Phe moiety showing that the nitrogen atom of the phenylalanine moiety bears the defined $R_1$ and $R_2$ moieties, and the ArgR$_3$ moiety indicating the OH radical of the carboxyl terminus of the arginine α-amino acid has been replaced with the $R_3$ moiety.

The compounds of this invention may be prepared by procedures analogously known in the art. In essence the synthesis of the fluorinated alkyl tripeptides of Formula I relies on a modified Dakin-West reaction of 2-phenyl-5(4H) oxazolone and the anhydrides of trifluoroacetic acid or pentafluoropropionic acid (depending on the desired $R_3$ moiety), to yield the requisite polyfluoro alkyl ketone amino acid derivatives for use as key intermediates. Further reactions then allow for the conversion of these amino acid analogs to the desired peptides of Formula I. These reactions are illustrated in the following reaction schemes:

**Reaction Scheme A**

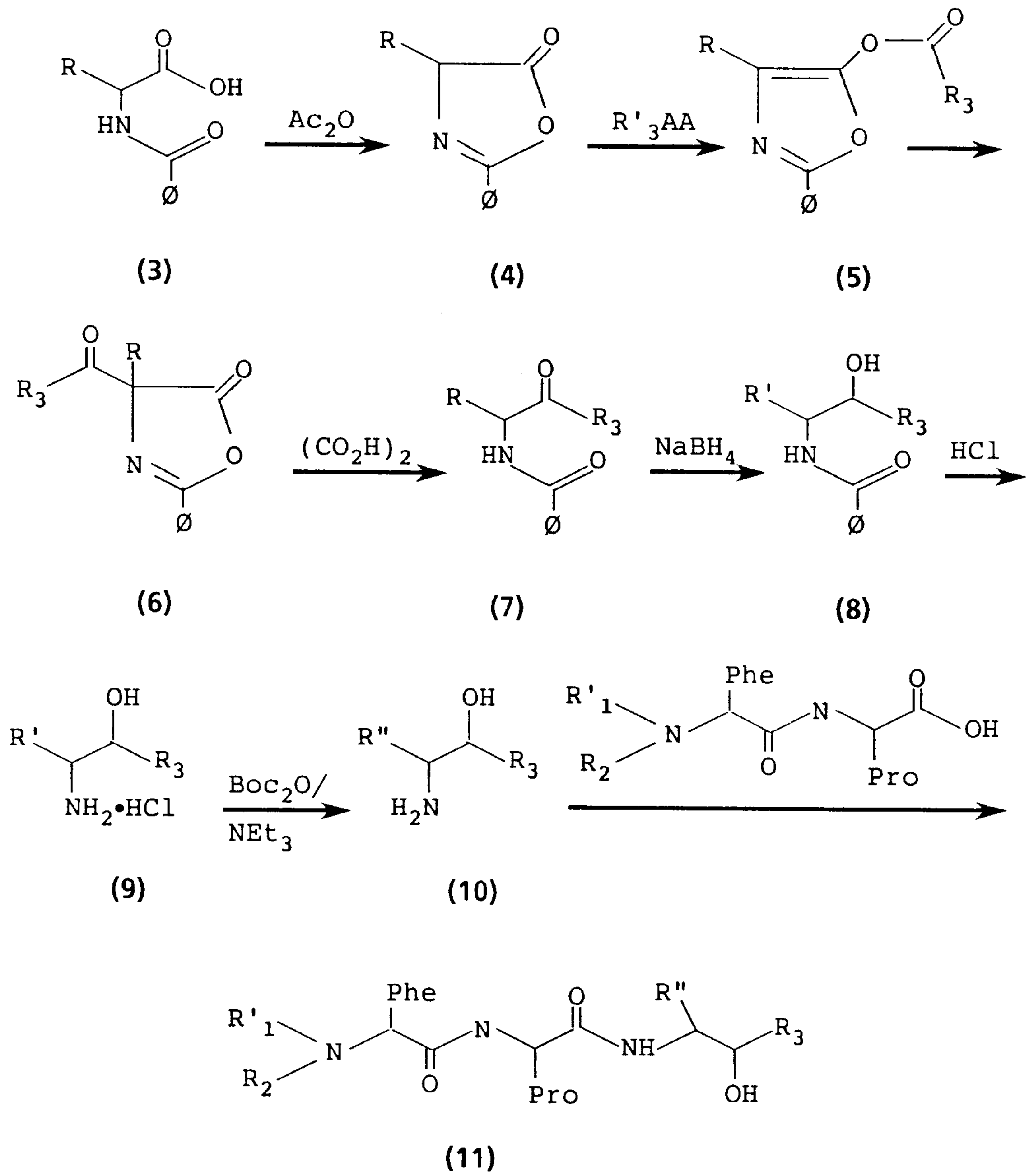


(3) (4) (5) (6) (7) (8) (9) (10) (11)

wherein $R_3$ is $CF_3$ or $CF_2CF_3$, R'$_3$AA are the anhydrides of trifluoroacetic acid or pentafluoropropionic acid,

Ø is phenyl, ØC(O)NH$(CH_2)_3$-, R' is HCl•$H_2N(CH_2)_3$-, R'' is Boc NH $(CH_2)_3$-, $R'_1$ is

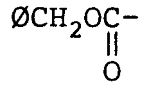

(which is also referred to as the Z amino protecting group), and $R_2$ is H or alkyl.

The N-benzoylated amino acid **(3)** is cyclized by standard techniques to the 5(4H)-oxazolone **(4)** ($Ac_2O$, 30 minutes, 90°C oil bath temperature) in good yield. Evaporation of the solvents affords a highly pure compound which can be used without further purification for the next step. Reaction of the oxazolone **(4)** with the appropriate polyfluoroacid anhydride is accomplished at 40°C (oil bath temperature) under an atmosphere of $N_2$ for 24 hours ($^1$H- and $^{19}$F-NMR-monitoring). When all of the starting material **(4)** is consumed, the C-4-acylated oxazolones **(5)** are the main products. Residual anhydride and polyfluorinated acids formed are removed under vacuum (0.01 torr; 25-70°C oil bath temperature, acetone/dry ice trap). Alternatively, Compound **(3)** may be converted to **(6)** by treatment with $R'_3$AA, and in such instances the intermediates are not isolated. The oily residues are then mixed with a saturated solution of freshly prepared anhydrous oxalic acid in tetrahydrofuran. (Commercial oxalic acid is dried for 16 hours at 100°C in a drying oven. Two subsequent sublimations (0.01 Torr, 90°C) afford anhydrous oxalic acid (m.p. 104°C) which is transferred under an atmosphere of $N_2$ into a flask and stoppered with a septum. Anhydrous tetrahydrofuran is added to the solid until most of it has dissolved (about 4 mL/g)) and the resulting solutions are stirred at room temperature for 16 hours, when gas evolution has completely ceased. Work-up (EtOAc/$H_2O$, aqueous $NaHCO_3$, brine; drying over $MgSO_4$) gives in satisfactory yield the desired fluorinated compounds **(7)** as mixtures of the ketones and their hydrated forms. Compounds **(7)** are targeted to (a) the transformation of the α-benzamido group of the ketones **(7)** to protected α-peptidylamino ketones and (b) the subsequent conversion of the δ-amino group of the ornithine analog **(7)** to a protected arginine analog.

For the intended transformation of the α-benzamides **(7)** the polyfluoroalkyl ketone functionality has to be temporarily masked. This can be achieved by reduction of the ketones **(7)** ($NaBH_4$;EtOH) to the alcohols **(8).** The benzamido function can now be cleaved by acid hydrolysis (6N HCl/AcOH). The second benzamido group is hydrolyzed under the reaction conditions and the diaminopolyfluoroalkyl alcohols **(9)** are isolated. Regioselective reprotection of the terminal amino group of the diamino alcohols **(9)** is accomplished with one equivalent of $Boc_2O$ and an excess of $NEt_3$ (4 mol. equiv.) in tetrahydrofuran/$H_2O$ to afford the Ω-Boc-protected diamino alcohols **(10).** Introduction of the protected peptidyl group to the 3-amino residue in the amino alcohols **(10)** can now be performed with a mixed anhydride of an $R'_1R_2$-PheProOH [Nicolaides, E., DeWald, H., Westland, R., Lipnik, M., and Posler, J., *J.Med.Chem.* 11, 74 (1968)] to give the completely protected tripeptide alcohol analogs of ornithine **(11).**

The final steps in the synthesis of the compounds of Formula I are depicted in Reaction Scheme B:

**Reaction Scheme B**

(11) 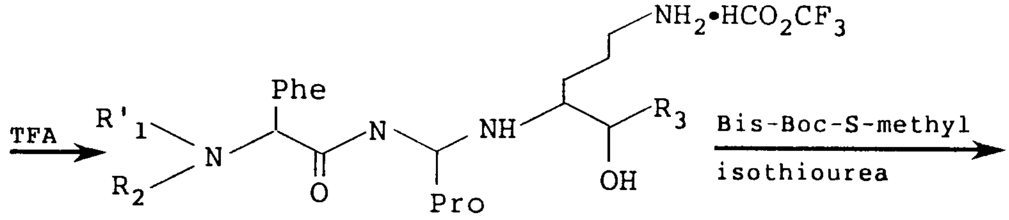


(12)

(13)

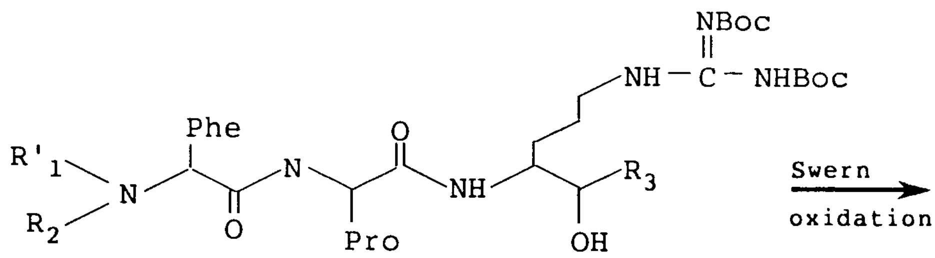


(14)

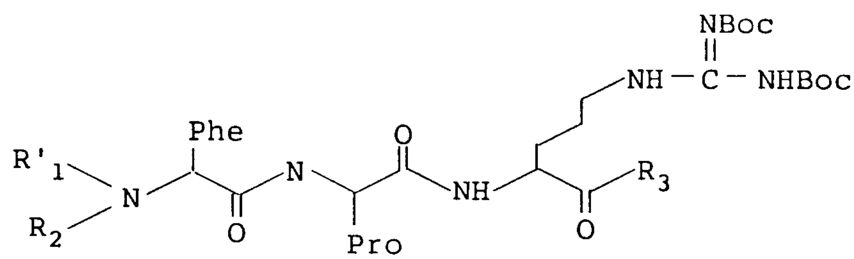

(15)

wherein TFA is trifluoroacetic acid, $R'_1$, $R_2$ and $R_3$ are as previously defined and Boc is the t-butoxycarbonyl amino protecting group.

The conversion of the ornithine analog (11) to a protected arginine tripeptide entails the deprotection of the side chain of the intermediate **(11)** using trifluoroacetic acid in methylene chloride to yield the corresponding aminohydrotrifluoroacetate salt **(12)** which is reacted with N,N'-bis-Boc-S-methylisothiourea **(13)** using 4 mol equivalents and triethylamine in anhydrous tetrahydrofuran to yield the desired protected arginine derivatives **(14)** which are oxidized using the well-known Swern oxidation procedures to produce intermediates **(15).** In the instance wherein $R_1$ is alkyl and $R_2$ is H (of Formula I) it is more convenient to use a bis Z-protected urea derivative, rather than the bis Boc urea derivative as a reactant.

Further processing of the oxidized intermediates **(15)** to the desired compounds of Formula I depends essentially on the definition of the $R_1$ and $R_2$ moieties. In those instances wherein it is desired to prepare final compounds wherein $R_1$ is H and $R_2$ is an alkyl, then the intermediates of Formula **(15)** to be utilized would be those wherein $R_1$ is lower alkyl. In such instances then $R'_1$ amino protecting group [i.e., Ø$CH_2$OC-(O)] and the two Boc-protecting groups would be removed by deprotection with liquid HF techniques well known in the art. In those instances wherein it is desired to prepare final compounds of Formula I wherein $R_1$ and $R_2$ are H, then the intermediates of Formula **(15)** to be utilized would be those wherein $R_2$ is H and again, both Boc-protecting groups on the arginine moiety and the Z-protecting group on the phenylalanine moiety would be removed by hydrogenation and by deprotection with HF. In those instances wherein it is desired to prepare final compounds of Formula I wherein $R_1$ and $R_2$ are both lower alkyl or $R_1$ and $R_2$ together with the nitrogen to which they are attached form a 5-6 membered heterocycle, then the intermediates of formula **(15)** to be utilized would be those wherein $R_2$ is H (and $R'_1$ is the Z-protecting group) then such intermediates would be reacted with the appropriate aldehyde (e.g., formaldehyde, glutaraldehyde or succinaldehyde) in the presence of $H_2/Pd(OH)_2$-C in isopropanol to form the desired N,N-dialkylated, pyrrolidine or piperidine derivatives. The remaining two Boc-protecting groups on the arginine moiety would be cleaved by treatment with HCl/$Et_2O$ or TFA in $CH_2Cl_2$ according to standard techniques well known in the art.

The following examples illustrate the preparation of the compounds of Formula I.

**EXAMPLE 1**

**N-Methyl-D-phenylalanyl-N[4-(aminoiminomethyl)amino-1-(trifluoroacetyl)butyl]-L-proline amide**

STEP A:

N,N'-bis-Benzoylornithine, ornithuric acid, **(1)**

A 250 ml flask, equipped with a magnetic stirring barr, two dropping funnels, and a pH-electrode was charged with 3.37 g (0.020 mol) of L-ornithine hydrochloride and 40 ml of N NaOH. The stirred solution was cooled to 0°C and solutions of benzoylchloride (5.0 ml, 0.044 mol) in diethylether (50 ml) and of N NaOH (44 ml) were added simultaneously at a rate to keep the pH at 12-13. Following complete addition the solution was allowed to warm to room temperature and stirred for further two hours. Diethylether (200 ml) was added, phases separated, and the aqueous layer acidified (pH 0-1, HCl conc.). The white precipitate formed was collected, washed with diethylether and dried (0.01 Torr) to afford 6.7 g (98%) of the title compound. m.p.: 184°C, (186°C[11]). $^1$H-NMR ($CDCl_3$, $CD_3OD$ 1:1): $\delta$ = 7.8 (m, 4H, benzoyl), 7.5 (m, 6H, benzoyl), 4.6 (m, 1H, CHN), 3.4 (m, 2H, $NCH_2$), 2.0-1.5 (m, 4H, $CH_2CH_2$).

STEP B:

N,N'-[1-(Trifluoroacetyl)-1,4-butanediyl]bis(benzamide), hydrate, **(2)**

A mixture of the ornithuric acid **(1)** (501.5 g, 1.47 mol) and TFAA (400 ml) was stirred at room temperature for one hour. The resulting solution was evaporated (0.1 Torr) and 1 L of TFAA was added. The solution was stirred for 16 hours and evaporated to dryness (0.01 Torr). Further 1 L of TFAA was added and the solution stirred for 4 hours. Reevaporation of the solution to dryness (0.1 Torr) was followed by addition of further 500 ml of TFAA. The solution was stirred for another 16 hours and evaporated to dryness (0.1 Torr). The oily residue was dissolved in 1.6 L of anhydrous THF and freshly dehydrated oxalic acid (440 g) added to the solution over a period of two hours. Stirring of the mixture was continued for 16 hours, when 7.5 L of ethyl acetate was added. The solution was washed with brine, aqueous sodium bicarbonate (4X), and brine, dried over $MgSO_4$ and evaporated to dryness. Yield of the title compound: 522.8 g (90.5%). Rf = 0.33 and 0.27 (eluent: ethyl acetate/petroleum ether 1:1), ketone and its hydrate.

m.p.: 113-115°C.

$^1$H-NMR ($CDCl_3$) $\delta$ = 8.0-7.5 (m, 4H, benzoyl), 7.5-7.0 (benzoyl, NH), 6.3 (m, 1H, NH), 5.0 [m, 0.5H, CH-(CO)$CF_3$], 4.5 [m, 0.5H, $CH(OH)_2CF_3$], 3.4 (m, 2H, $NCH_2$), 2.2-1.6 (m, 4H, $CH_2CH_2$).

$^{19}$F-NMR ($CDCl_3$) $\delta$ = 86.00 (s, $COCF_3$), 80.00 [s, $CF_3C(OH)_2$]; ratio 1:1, ketone and hydrate.

MS (m/e): 393 ($MH^+$/30% rel. intensity).

| Analysis calculated for $C_{20}H_{19}F_3N_2O_3$ $H_2O$ (410.40): | | | |
|---|---|---|---|
| | C: 58.53; | H: 5.16; | N: 6.83. |
| Found: | C: 58.62; | H: 5.22; | N: 6.64. |

STEP C:

N,N'[1-(2,2,2-Trifluoro-1-hydroxyethyl)-1,4-butanediyl]-bis(benzamide), **(3)**

$NaBH_4$ (45 g, 1.2 mol) was added portionwise to a cooled (0°C) and stirred solution of the trifluoromethyl ketone **(2)** (522 g, 1.33 mol) in 6.5 L of EtOH. The solution was stirred for 10 minutes at 0°C and then at room temperature for 16 hours. Aqueous HCl (6N) was added and, when effervescence had stopped, solid sodium carbonate was added until the pH of the solution was basic. AcOEt was added and phases separated. The aqueous layer was extracted twice with AcOEt and the combined organic phases

washed with brine. Drying of the organic solution ($MgSO_4$) and flash-evaporation (20 Torr, 30 °C) afforded an oil which solidified upon standing. Yield 457.6 g (87%).
m.p.: 103 °C, Rf = 0.7 (AcOEt).
$^1$H-NMR ($CDCl_3$/$CD_3OD$ 1:1): δ = 7.9 (m, 4H, benzoyl), 7.4 (m, 6H, benzoyl), 4.4 (m, 1H, CHN), 4.1 (m, 1H, $CHCF_3$), 3.3 (m, 2H, $NCH_2$), 2.0-1.5 (m, 4H, $CH_2CH_2$).
$^{19}$F-NMR ($CDCl_3$/$CD_3OD$ 1:1) δ = 87.33 and 85.83 (2d, $J_{HF}$ = 7.5 Hz); ratio 3:1.
A small sample (100 mg) was allowed to crystallize for CHN analysis.

| Analysis calculated for $C_{20}H_{21}F_3N_2O_3$ (394.401): | | | |
|---|---|---|---|
| | C: 60.76; | H: 5.52; | N: 7.05. |
| Found: | C: 60.55; | H: 5.55; | N: 7.01. |

STEP D:

3,6-Diamino-1,1,1-trifluoro-2-hexanol, dihydrochloride, **(4)**

A stirred solution of the N,N'-bis-protected diaminoalcohol **(3)** (457 g) in aqueous HCl 12N (5 L) was heated for 16 hours to reflux. Solvents were evaporated and the oily residue dissolved in $H_2O$. The aqueous solution was washed with diethylether and evaporated to dryness to afford 260 g (87% yield) of the trifluorodiaminoalcohol, hydrochloride as a slightly colored oil.
$^1$H-NMR ($D_2O$): δ = 4.6 (m, 1H, $CHCF_3$), 3.8 (m, 1H, CHNH), 3.2 (m, 2H, $NCH_2$), 2.0 (m, 2H, $CH_2CH_2$).
$^{19}$F-NMR ($D_2O$, $C_6F_6$ as ext. ref.): δ = 87.3 and 84.8 (2d, $J_{HF}$ = 7.5 HZ); ratio 3:1.

STEP E:

(4-Amino-6,6,6-trifluoro-5-hydroxyhexyl)carbamic acid,1,1-dimethylethyl ester, **(5)**

Di-*tert*-butyldicarbonate (217 g, 0.99 mol) was added portionwise to a cooled (-5 °C) and well stirred solution of the trifluoromethyl-diamino alcohol, dihydrochloride **(4)** (260 g, 1.0 mol) and $NEt_3$ (>4 equiv, ca. 600 ml) in THF/$H_2O$ (3 L/3 L). Stirring was continued after complete addition for 1 hour at 0 °C and 16 hours at room temperature. The solution was evaporated to one half of its original volume, acidified (solid citric acid), washed with diethylether, basified (NaOH pellets, pH 12-13), and exhaustively extracted with diethylether. The combined etheral extracts were washed with brine, dried ($MgSO_4$) and evaporated (20 Torr, 30 °C) to afford the Ω-protected diamino alcohol (5).
$^1$H-NMR ($CDCl_3$) δ = 4.8 (m, 1H, CHN), 4.0-3.5 (m, 1H, $CHCF_3$), 3.2 (m, 2H, $NCH_2$), 2.5 (m, 3H, exchange with $D_2O$, OH, $NH_2$), 1.8-1.3 (m, 4H, $2CH_2$), 1.45 (s, 9H, $3CH_3$).
$^{19}$F-NMR ($CDCl_3$): δ = 88.33 and 84.33 (2d, $J_{HF}$ = 7.5 Hz); ratio 3:1.
An aliquot (100 mg) was treated with diethylether/methanol (10:1), the solid collected and dried to afford 60 mg of anlytically pure **(5).**

| Analysis calculated for $C_{11}H_{21}F_3N_2O_3$ (286.301): | | | |
|---|---|---|---|
| | C: 46.15; | H: 7.39; | N: 9.78. |
| Found: | C: 46.25; | H: 7.62; | N: 9.46. |

STEP F:

N-Methyl-N-(phenylmethoxycarbonyl)-D-phenylalanyl-N-[4-(1,1-dimethylethoxycarbonylamino]-1-[(2,2,2-trifluoro-1-hydroxyethyl)butyl]-L-proline amide, **(6)**

Isobutylchloroformate (2.46 ml, 18.94 mol) was added under an atmosphere of $N_2$ over a period of 10 minutes to a cooled (-20 °C) and stirred solution of N-methyl-N-phenylmethyloxycarbonyl-D-phenylalanyl-L-proline (7.06 g, 17.22 mol) (S Bajusz et al., J. Med. Chem., 1990, 33, 1729) and N-methylmorpholine (2.08 ml, 18.94 mol) in anhydrous THF (50 ml). The reaction mixture was stirred for 1 hour at 20 °C and a

solution of the Ω-N-protected diaminoalcohol **(5)** (4.92 g, 17.22 mol) in THF (anhydrous, 50 ml) was added. The mixture was stirred for 1 hour at 0°C and then kept for 16 hours at +4°C. The solvent was evaporated, the residual oil dissolved in AcOEt/$H_2O$, and phases separated. The organic layer was washed with saturated solutions of $NaHCO_3$, citric acid, and brine, dried ($MgSO_4$), and evaporated. The resulting crude tripeptide derivative (15 g, yellow foam) was applied to flash chromatography on silica gel (eluent: AcOEt/hexane 1:1) and the combined product containing fractions were evaporated (20 Torr, 30°C; twice with $CCl_4$ as cosolvent, then 0.01 Torr at room temperature, 16 hours) to afford 11.68 g (62%) of the title compound as a colorless foam.
Rf= 0.24 (AcOEt/hexane 2:1).
$^1$H-NMR (90 MHz, $CDCl_3$): δ= 7.4-7.2 (2m, 10H, 2 aryl), 7.1 (m, 1H, NH), 6.9 (m, 1H, NH), 5.8-5.5 (m, 2H, 2NH), 5.1 (m, 2H, arylCHO$_2$), 4.8-3.5 [6m, 5H, 4CH, OH (exchanges with $D_2O$)], 3.4-2.6 (m, $NCH_3$, $2NCH_2$, aryl$CH_2$), 2.2 and 1.9-1.5 (m, 8H, $4CH_2$), 1.4 (m, 9H, $3CH_3$).
$^{19}$F-NMR ($CDCl_3$): δ= 87.1-86.8 (m).

STEP G:

N-Methyl-N-(phenylmethoxycarbonyl)-D-phenylalanyl-N-[4-amino-1-(2,2,2-trifluoro-1-hydroxyethyl)butyl]-L-proline amide, trifluoroacetate (salt) hydrate, **(7)**

A solution of 25 ml of TFA in 25 ml of $CH_2Cl_2$ was added to the protected tripeptide derivative **(6)** (7.0 g, 10.32 mol) and the mixture was stirred until effervescence had stopped (30 minutes, bubble counter). The solution was evaporated to dryness (0.01 Torr, 40 hours) to afford a yellow foam. The title compound was precipitated from i.-propanol/petroleum ether to give, after drying, 7.05 g (yield: quantitative) of a white solid.
Rf= 0.5 (AcOEt/10% AcOH).
$^1$H-NMR (90 MHz, $CD_3OD$) δ= 7.3 (m, 10H, 2 phenyl), 5.1 (m, 2H, $CH_2O$), 4.6-4.45 (m, 1H, CHN-phe), 4.2 (m, 1H, CHN-pro), 4.1 (m, 1H, CHN), 3.9 (m, 1H, $CHCF_3$), 3.1-2.6 (m, 9H, $NCH_3$, $3NCH_2$), 2.0-1.3 (m, 8H, $4CH_2$).
$^{19}$F-NMR (90 MHz, $CD_3OD$): δ= 88.5 (m, $CF_3CO_2H$, $CF_3$).

| Analysis calculated for $C_{30}H_{38}F_6N_{40}7H_{20}$ (710.66): | | | |
|---|---|---|---|
| | C: 52.38; | H: 5.60; | N: 7.49. |
| Found: | C: 52.59; | H: 5.60; | N: 7.49. |

STEP H:

N-Methyl-N-(phenylmethoxycarbonyl)-D-phenylalanyl-N-[4-[N',N''-bis[(1,1-bis(phenylmethoxycarbonyl)[-(aminoiminomethyl)amino]]-1-[(2,2,2-trifluoro-1-hydroxyethyl)butyl]-L-proline amide, hemihydrate, **(8)**

N,N'-bis-Benzyloxycarbonyl-S-methylisothiourea (3.46 g, 9.7 mol) was added under an atmosphere of $N_2$ to a solution of the trifluoroacetate salt **(7)** (5.57 g, 8 mol) and $NEt_3$(1.67 ml), in 50 ml of anhydrous THF. The solution was stirred at 40°C for 4 days. THF was evaporated and the solid residue dissolved in $Et_2O$, The etheral solution was washed with a saturated solution of citric acid and brine, dried ($MgSO_4$), and solvents evaporated. The solid residue was applied to a flash chromatography column charged with $SiO_2$ -(230-400 mesh, eluent AcOEt/hexane 1:2) and when all residual isothiourea (Rf= 0.8) had sorted AcOEt/hexane 1:1-2:1 and the combined product containing fractions were evaporated to afford 5.08 g (71% yield) of the guanidino tripeptide derivative (13) as a colorless foam.
Rf= 0.3 (AcOEt/hexane 2:1).
$^1$H-NMR (360 MHz, $CDCl_3$): δ= 11.5 (s, broad, 1H, ZNH guanidine), 8.3 (m, 1H, NH guanidine), 7.3-7.2 (m, 22H, 4 phenyl, 2NH), 5.7 (m, 1H, NH), 5.1 (m, 7H, NH, $3CH_2O$), 4.8-3.9 (m, 5H, CHN-phe, $CF_3CHO$, CHN-pro, CHN, NH), 3.8-2.5 (m, 9H, $NCH_2$-pro, $NCH_2$ guanidine $NCH_3$, $CH_2$-phe), 1.8-1.5 (m, 8H, $4CH_2$).
$^{19}$F-NMR (360 MHz, $^1$H-decoupled, $CDCl_3$): δ= 87.49, 87.12, 87.00 and 85.16 (4s).

| Analysis calculated for $C_{46}H_{51}F_3N_6O_9 \cdot 0.5\ H_2O$ (897.96): | | | |
|---|---|---|---|
| | C: 61.53; | H: 5.84; | N: 9.36. |
| Found: | C: 61.53; | H: 5.75; | N: 9.27. |

STEP I:

N-Methyl-N-(phenylmethoxycarbonyl)-D-phenylalanyl-N-[4-N',N''-bis[phenylmethoxycarbonyl-(aminoiminomethyl)amino]-1-[(2,2,2-trifluoroacetyl)butyl]-L-proline amide, dihydrate, **(9)**

A 250 ml three necked flask, equipped with a magnetic stirring barr, thermometer, and an $N_2$ inlet was charged with a solution of oxalylchloride (0.74 ml, 8.4 mol) in 10 ml of anhydrous $CH_2Cl_2$ and placed in a dry-ice/acetone bath (-55 °C internal temperature). A solution of DMSO (1.2 ml, 17 mol) in 200 ml of $CH_2Cl_2$ was added under an atmosphere of $N_2$ at a rate to keep the internal temperature at -55 °C. Stirring was continued for 5 minutes and a solution of the alcohol **(8)** (4.98 g, 5.6 mol) in 10 ml of $CH_2Cl_2$ was added in one portion. The mixture was allowed to warm to -25 °C and stirred at that temperature for 10 minutes. The solution was cooled again to -55 °C, 3.9 ml of $NEt_3$ is added slowly, and the ice bath removed. When the internal temperature had reached -20 °C a saturated solution of citric acid was added. 250 ml of $CH_2Cl_2$ were added at room temperature, phases separated and the organic layer washed with $H_2O$, a saturated solution of $NaHCO_3$, and brine. Drying ($MgSO_4$) and evaporation of the solvents afforded 5.07 g of a yellow oil. Flash chromatography on $SiO_2$ (230-400 mesh, eluent AcOEt/hexane 1:1) and evaporation of the combined product containing fractions gave 3.71 g (74%) of the ketone as a colorless foam.
Rf = 0.5 (AcOEt/hexane 2:1).
$^1$H-NMR ($CDCl_3$): $\delta$ = 11.5 (broad s, 1H, NH-Z), 8.5 (m, 1H, NH guanidine), 7.3 (m, 20H, 4 phenyl), 5.7 (3m, 1H, NH), 5.1 (m, 6H, $3CH_2O$), 4.8 (m) and 4.7-4.2 (m, 4H, 4CH), 3.9 (m) 3.7-3.3 (m) and 2.6 (m, 9H, $NCH_2$, pro, $NCH_2$ guanidine, $NCH_3$, aryl$CH_2$), 2.0-1.5 (m, 8H, $4CH_2$).
$^{19}$F-NMR ($CDCl_3$): $\delta$ = 86.08, 85.96, 85.88 and 85.83 (4s).

| Analysis calculated for $C_{46}H_{49}F_3N_6O_9 \cdot H_2O$ (913.95): | | | |
|---|---|---|---|
| | C: 60.45; | H: 5.74; | N: 9.20. |
| Found: | C: 60.52; | H: 5.56; | N: 9.16. |

STEP J:

N-Methyl-D-phenylalanyl-N[4-(aminoiminomethyl)amino-1-(trifluoroacetyl)butyl]-L-proline amide, **(10)**

A solution of the ketone **(9)** (2.94 g, 3.3 mol) in 100 ml of isopropanol, 15 ml of HCl 1N, and 0.5 g of $Pd(OH)_2$ catalyst was hydrogenated under atmosphere pressure for 4 days. Evaporation of the solvent left an oily residue which was dissolved in water. The aqueous phase was washed with ether and lyophilized to give 665 mg (62% yield) of the title compound as a white powder.
Rf = 0.5, AcOH/BuOH/$H_2O$ 1/3/1.
$^1$H-NMR ($D_2O$): $\delta$ = 7.4 and 7.3 (2m, phenyl), 4.45, 4.35 and 4.2 [3m, 2H, NCHCO, $CHC(OH)_2CF_3$], 3.5-3.1 and 2.8 (3m, 6H, $NCH_2$ guanidine, $NCH_2$-pro, $CH_2$-phe, $NCH_3$), 2.2-1.4 (m, 8H, $4CH_2$), 2.0 (s, $CH_3CO_2H$).
$^{19}$F-NMR ($D_2O$): $\delta$ = -6.16 and -6.34 [2s, ratio 55.45, $CF_3C(OH)_2$].

By substituting the TFAA reactant used in Step B of this example with equivalent quantities of pentafluoropropionic anhydride (PFPAA), and by substantially following the procedures of Steps B to J, there is produced N-methyl-D-phenylalanyl-N-[4-aminoiminomethyl)amino-1-pentafluoropropionyl)butyl-L-proline amide.

**EXAMPLE 2**

**D-Phenylalanyl-N[4-(aminoiminomethyl)amino]-1-(trifluoroacetyl)butyl]-L-prolineamide, 1,5 HF, 0.5 $CH_3CO_2H$, $3H_2O$**

STEP A:

N-(Phenylmethoxycarbonyl)-D-phenylalanyl-N-[4-(1,1-dimethylethoxycarbonylamino] -1-[2,2,2-trifluoro-1-hydroxyethyl)butyl]-L-prolineamide, **(11)**

Isobutylchloroformate (450 µl, 346 mmol) is added under an atmosphere of $N_2$ to a cooled (-10 ° C) and stirred solution of Phenylmethyloxycarbonyl-D-phenylalanyl-L-proline (Z-D-Phe-Pro-OH, 1.27 g, 3.2 mmol) and N-methylmorpholine (380 µl, 3.46 mmol). The reaction mixture is stirred for 10 minutes at -10 ° C and a solution of the Ω-N-protected diaminoalcohol of Step E of Example 1 (900 mg, 3.19 mmol in tetrahydrofuran (anhydrous, 6 ml) is added. The mixture is stirred for 1 hour at 0 ° C and then kept for 16 hours at +4 ° C. AcOEt/$H_2O$ (100 ml each) is added and phases separated. The organic layer is washed with saturated solutions of $NaHCO_3$, citric acid, and brine (each 3 × 50 ml), dried ($MgSO_4$), and evaporated. The resulting crude tripeptide derivative (2.5 g, yellow foam) is applied to flash chromatography ($SiO_2$, 100 g, eluent: AcOEt/hexane 1:1) and the combined product containing fractions are evaporated (20 Torr, 30 ° C; twice with $CCl_4$ as cosolvent, then 0.01 Torr, room temperature, 16 hours) to afford 1.5 g (72%) of the title compound as a colorless foam.
Rf = 0.14 (AcOEt/hexane)
$^1H$ NMR (360 MHz, $CDCl_3$) δ = 7.4-7.2 (3m, 10H, 2 aryl), 7.1 (m, 1H, NH), 5.8-5.5 (m, 2H, 2NH), AB centered at 5.10 ($J_{HA\text{-}HB}$ = 10 Hz, 2H, aryl$CH_2O$), 4.8-3.5 [6m, 5H, 4CH, OH (exchanges with $D_2O$)], 3.2-2.9 and 2.6 (2m, 6H, 2N$CH_2$, aryl$CH_2$), 2.2 and 1.9-1.5 (m, 8H, 4$CH_2$), 1.4 (m, 9H, 3$CH_3$).
$^{19}F$-NMR ($CDCl_3$): δ = 87.1; 86.8 (2d, $J_{HF}$ = 7.5 Hz), 86.5 (m), 85.2; 85.1 (2d, $J_{HF}$ = 7.5 Hz), 85.0 (m).
MS (CI/$NH_3$): m/e = 665 ($MH^+$, 50%).
Anal. calcd. for $C_{33}H_{43}F_3N_4O_7 \cdot 0.4\ CCl_4$ (726.258): C 54.94, H 5.97, N 7.71. Found C 54.74, H 6.12, N 7.29.

Step B:

N-[(Phenylmethoxy)carbonyl]-D-phenylalanyl-N-[4-amino-1-(2,2,2-trifluoro-1-hydroxyethyl)butyl]-L-prolineamide, trifluoroacetate (salt), hemihydrate, **(12)**

A solution of 5 ml TFA in 5 ml of $CH_2Cl_2$ is added to the protected tripeptide derivative (1.39 g, 2.09 mol) and the mixture is stirred until effervescence has stopped (20 min, bubble counter). The solution is evaporated to dryness (0.01 Torr, 40 hours). An aliquot (680 mg) was taken from the resulting yellow foam, 100% for the following reaction, and the rest treated with i-propanol/hexane to afford 730 mg of the title compound as a white solid.
Rf = 0.5 (AcOEt/10% AcOH).
$^1H$-NMR (360 MHz, $CD_3OD$): δ = 7.3 (m, 10H, 2 phenyl), AB's centered at 5.1 ($J_{HA\text{-}HB}$ = 23 Hz, 2H, $CH_2O$), 4.6 (t, J = 7.5 Hz, 0.5H), and 4.45 (dd, J = 7.5 Hz, 0.5H, 1H, $CHCF_3$), 2.9 and 2.6 (2m, 6H, 3N$CH_2$), 2.0-1.3 (m, 8H, 4$CH_2$).
$^{19}F$-NMR (360 MHz, $CD_3OD$): δ = 88.48 (s, $CF_3CO_2H$), 88.35, 88.20, 87.21, and 87.18 (4d, $J_{HF}$ = 7 Hz, $CF_3$); ratio 16:10:3:3.
MS (CI/$NH_3$): m/e = 565 (MH+, 100% rel. intensity).
Anal. calcd. for $C_{30}H_{36}F_6N_4O_7 \cdot 0.5\ H_2O$ (687.64): C 52.40, H 5.42, N 8.15. Found C 52.30, H 5.69, N 7.83.

Step C:

N-[(Phenylmethoxy)carbonyl]-D-phenylalanyl-N-[4-[N',N''-bis[(1,1-dimethylethoxy)carbonyl][-(aminoiminomethyl)amino]]-1-[(2,2,2-trifluoro-1-hydroxyethyl)butyl]-L-prolineamide, hydrate, **(13)**

N,N'-Bis-Boc-S-methylisothiourea [Bergeron, R.J. and McManis, J.S., *J.Org.Chem.*, **1987,** 52, 1700] (1.40 g, 4.83 mmol) is added under an atmosphere of $N_2$ to a solution of the trifluoroacetate salt **(12)** (820 mg, 1.2 mmol) and $NEt_3$ (250 µl, 1.81 mmol) in 4 ml of anhydrous tetrahydrofuran. The solution is stirred at 40 ° C for 4 days. Tetrahydrofuran is evaporated and the solid residue dissolved in $Et_2O$. The ethereal solution is washed with a saturated solution of citric acid and brine, dried ($MgSO_4$), and solvents evaporated. The solid residue is applied to a flash chromatography column charged with $SiO_2$ [30 g, 230-400 mesh, eluent AcOEt/hexane 1:2 and when all residual isothiourea (Rf = 0.8) has sorted AcOEt/hexane 1:1-2:1] and the combined product containing fractions are evaporated to afford 440 mg (45%) of the quanidino tripeptide derivative (13) as a colorless foam.
Rf = 0.3 (AcOEt/hexane: 2:1).

$^1$H-NMR (360 MHz, $CDCl_3$): $\delta$ = 11.5 (s, broad, 1H, BocNH guanidine), 8.3 (m, 1H, NH guanidine), 7.2 (m, 11H, 2 phenyl, NH), 6.0, 5.9, and 5.7 (3m, 1H, NH), AB's centered at 5.1 (J = 23 Hz, 2H, $CH_2O$), 4.4 (m, 1H, CHN phe), 4.3-3.2 (6m, 7H, CHN pro, $NCH_2$ pro, $NCH_2$ guanidine A-part, $CHCF_3$, OH), 3.0 (m, 2H, aryl $CH_2$), 2.6 (m, 1H, $NCH_2$ guanidine B-part), 1.8-1.2 (m, 26H, $6CH_3$, $4CH_2$).
$^{19}$F-NMR (360 MHz, $CDCl_3$): $\delta$ = 86.95, 86.86, 85.39, and 85.16 (4d, $J_{HF}$ = 7 Hz); ratio 5:5:1:1.
Anal. calcd. for $C_{39}H_{43}F_3N_9O_6 \cdot H_2O$ (824.88): C 56.79, H 6.72, N 10.19. Found C 56.53, H 6.54, N 10.05.

Step D:

N-[(Phenylmethoxy)carbonyl]-D-phenylalanyl-N-[4-[N',N''-bis[(1,1-dimethylethoxy)carbonyl][-(aminoiminomethyl)amino]]-1-[(2,2,2-trifluoroacetylbutyl]-L-prolineamide, dihydrate, **(14)**

A 25 ml three necked flask, equipped with a magnetic stirring barr, thermometer, and an $N_2$ inlet is charged with a solution of oxalylchloride (127 mg, 1 mmol) in 1 ml of anhydrous $CH_2Cl_2$ and placed in a dry ice/acetone bath (-55 °C internal temperature). A solution of DMSO (220 mg, 2.8 mmol) in 1 ml of $CH_2Cl_2$ is added under an atmosphere of $N_2$ at a rate to keep the internal temperature at -55 °C. Stirring is continued for 5 minutes and a solution of the alcohol **(13)** (400 mg, 0.5 mmol) in 2 ml of $CH_2Cl_2$ is added in one portion. The mixture is allowed to warm to -25 °C and stirred at that temperature for 10 minutes. The solution is cooled again to -55 °C, 0.5 ml of $NEt_3$ is added slowly, and the ice bath removed. When the internal temperature has reached -20 °C a saturated solution of citric acid is added. 50 ml of $CH_2Cl_2$ are added at room temperature, phases separated and the organic layer washed with water, a saturated solution of $NaHCO_3$, and brine. Drying ($MgSO_4$) and evaporation of the solvents affords 340 mg of a yellow oil. Flash chromatography on $SiO_2$ (10 g, 230-400 mesh, eluent AcOEt/hexane 1:1, then 2:1) and evaporation of the combined product containing fractions gives 240 mg (60%) of the ketone **(14)** as a colorless foam.
Rf = 0.5 (AcOEt/hexane: 2:1).
$^1$H-NMR ($CDCl_3$): $\delta$ = 11.5 (s, broad, 1H, BocNH), 8.5 (m, 1H, NH guanidine), 7.3 (m, 10H, 2 phenyl), 5.7 (3m, 1H, NH), 5.1 (m, 2H, $CH_2O$), 4.8 (m) and 4.7-4.2 (m, 4H, 4CH), 3.9 (m) 3.7-3.3 (m) and 2.6 (m, 4H, $NCH_2$ pro, $NCH_2$ guanidine), 3.0 (m, 2H, aryl $CH_2$), 2.0-1.4 (m, including s at 1.5, 26H, $6CH_3$, $4CH_2$).
$^{19}$F-NMR ($CDCl_3$, H-decoupled): $\delta$ = 86.14 and 85.89 (2d, ratio 1:2, $CF_3CO$), 80.22 and 79.69 [2s, ratio 1:1, $CF_3C(OH)_2$].
$^{13}$C-NMR ($CDCl_3$): $\delta$ = 175.5-174.29 (4s, CONH), 164.1 (2s), 158.8-156.8 (4s) and 153.9 (s, OCON, OCON=C), 136.5 and 136.2 (2s, phenyl), 130.8-128.0 (8s, phenyl), 125.57, 122.38, and 121.19 (visible part of q, $J_{CF}$ = 107.6 Hz, $CF_3$), 95.4-94.7 [4s, $C(OH)_2$], 83.8-83.7 (3s) and 80.0, 79.9 (2s, C), 68.2-67.8 (3s, $CH_2O$), 61.5-59.4 (2s, NCH pro), 55.6-54.4 (several s, NCH phe, NCH $COCF_3$), 47.87 and 47.65 (2s, $NCH_2$ pro), 41.0-40.7 (2s, $NCH_2$ guanidine), 39.11-38.48 (2s, phenyl $CH_2$), 31.8-24.6 (several s, $CH_2$ pro, 2-*tert.*-butyl, $CH_2$ guanidine, $CH_2$ pro).
Anal. calcd. for $C_{39}H_{51}F_3N_6O_9 \cdot 2H_2O$ (840.86): C 55.71, H 6.59, N 9.99. Found C 55.75, H 6.25, N 9.78.

Step E:

D-Phenylalanyl]-N-[4-(aminoiminomethyl)amino]-1-(trifluoroacetylbutyl]-L-prolineamide,1.5 HF, 0.5 $CH_3CO_2H$, $3H_2O$

The reaction flask of a 'Sakakibara apparatus" is charged with 100 mg (0.119 mmol) of the ketone of Step D and applied to HF deprotection of all protecting groups. After a reaction time of 20 minutes HF is evaporated, the solid residue dissolved in a mixture of AcOH/$H_2O$ 1:1, and lyophilized. The solid residue is dissolved in water, the mixture filtered through a Millipore filter, and lyophilized again to afford 40 mg (57%) of the title compound as a solid.
$^1$H-NMR ($D_2O$): $\delta$ = 7.4 and 7.3 (2m, phenyl), 4.45, 4.35 and 4.2 [3m, 2H, NCHCO, $CHC(OH)_2CF_3$], 3.5-3.1 and 2.8 (3m, 6H, $NCH_2$guanidine, $NCH_2$ pro, $CH_2$ phe), 2.2-1.4 (m, 8H, $4CH_2$), 2.0 (s, $CH_3CO_2H$).
$^{19}$F-NMR ($CD_2O$): $\delta$ = -2.91 (s, $CF_3CO$), -6.16 and -6.34 [2s, ratio 55:45, $CF_3C(OH)_2$], -54.28 (s, $F^-$ = 1.5 HF/$CF_3$.
Anal. calcd. for $C_{21}H_{29}F_3N_6O_3 \cdot 1.5\ HF \cdot 0.5\ CH_3CO_2H \cdot 3\ H_2O$ (584.58): C 45.20, H 6.64, N 14.38. Found C 55.75, H 6.25, N 9.78.

By substituting the TFAA reactant of Step B of Example 1 with equivalent amounts of PFPAA and by following Steps B, C, D, E of Example 1 and using the product of that reaction in Step A of this example and by following the teachings of Steps B, C, D and E of this example there is produced D-phenylalanyl-N-[4-(aminoiminomethyl)amino]-1-(pentafluoropropionylbutyl]-L-prolineamide, 1.5 HF, O.5 $CH_3CO_2H$, $3H_2O$.

## EXAMPLE 3

**N,N-Dimethyl-D-phenylalanyl-N[4-aminoiminomethyl)amino]-1-(trifluoroacetyl)butyl]-L-prolineamide, dihydrochloride, hydrate**

STEP A:

N,N-Dimethyl-D-phenylalanyl-N-[4-[N',N''-bis[(1,1-dimethylethoxy)carbonyl][(aminoiminomethyl)amino]]-1-[-(2,2,2-trifluoroacetyl)butyl]-L-prolineamide, hydrate

An aqueous solution of formaldehyde (37%, 24 µl) is added to a suspension of the fully protected ketone (product of Step D, Example 2, 822 mg, 1 mmol) in isopropanol (10 ml) and 200 mg of the catalyst $Pd(OH)_2$ on carbon. The resulting suspension is hydrogenated under atmospheric pressure until hydrogen uptake is stopped (≃ 24 hours). Filtration from the catalyst affords a slightly yellow solutionwhich is evaporated to one third of its original volume. The residue is diluted with 10 ml of water and the solution lyophilized to give a yellow foam which is used as such in the next step.

Step B:

N,N-Dimethyl-D-phenylalanyl-N[4-aminoiminomethyl)amino]-1-(trifluoroacetyl)butyl]-L-prolineamide, dihydrochloride, hydrate

To a suspension of the above-prepared ketone (720 mg, 1 mmol) in anhydrous ether (10 ml) is added a saturated solution of HCl-gas in anhydrous ether (4 M, 50 ml). Stirring of the mixture for 2 days and evaporation of the solvents afford the title compound as a slightly yellow foam (600 mg).
Rf = 0.5, ($BuOH/AcOH/H_2O$ 3/1/1).

By substituting the formaldehyde reactant of Step A of Example 3 with equivalent amounts of succinaldehyde or glutaraldehyde and by following the processes of Steps A and B, there is produced the pyrrolidine and piperidine analogs, respectively, of the dimethyl-D-Phe compound of Example 3, (i.e., the corresponding salts of

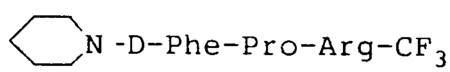

and

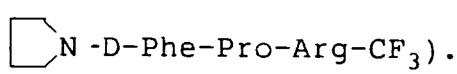

Similarly, by substituting the trifluoroacetic anhydride (TFAA) of Step B of Example 1 with equivalent amounts of pentafluoropropionic anhydride (PFPAA) and by following the teachings of Examples 1-3 there is produced the corresponding pentafluoropropionyl analogs [i.e., the salts of compounds

(a) $(CH_3)_2N$-D-Phe-Pro-Arg-$CF_2CF_3$,

(b)

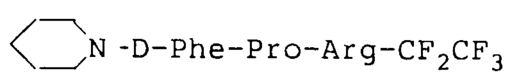

and

(c)

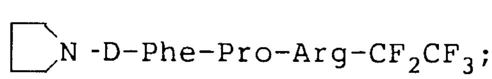

it being noted that the nitrogen atom of the depicted pyrrolidine and piperidine moieties is the terminal nitrogen atom of the D-phenylalanine α-amino acid.

Using standard *in vitro* assay methodology for determining inhibitory activity of thrombin as well as anticoagulant activities in human plasma using activated thromboplastin times (aPTT) and thrombin times (TT), it is to be found that the compounds of Formulae I and XX are effective anticoagulants. Anticoagulant data may be represented as follows:

| ANTICOAGULANT EFFECT IN HUMAN PLASMA | | |
|---|---|---|
| Compound | $ID_2$[a] (μM) | |
| | aPTT[b] | TT[b] |
| D-$CH_3$Phe-Pro-Arg-$CF_3$ | 218 | 108 |
| D-Phe-Pro-Arg-$CF_3$ | 148 | 43 |
| D-Phe-Pro-Arg-$CF_2CF_3$ | 2 | 1 |

[a]Amount required for doubling the clotting time;
[b]aPTT, activated partial thromboplastin time;
TT, thrombin time.

*In vivo* anti-thrombotic effects in rats may also be utilized for evaluation. For example, in the $FeCl_3$ -induced thrombosis model wherein D-Phe-Pro-Arg-$CF_2CF_3$ was administered intravenously as a 10 mg/kg of body weight injection, followed by a continuous 1 mg/kg/minute infusion, the compound was capable of preventing occlusion in two rats and prolonging occlusion in the other 2 of the 4 rats. Thus, based upon standard *in vitro* and *in vivo* assay methodology it is to be found that the compounds of this invention, at doses within the range of about 5 to 50 mg per kilogram of body weight, per day will be useful in the treatment of thrombophlebitis and coronary thrombosis. Of course, actual dosage and frequency will vary dependent upon the nature and severity of the condition, age, general health conditions and such other factors well known and appreciated by the skilled attending diagnostician.

In addition to the foregoing use of the novel compounds of Formula I as inhibitors of thrombin, another aspect of this invention is the use of the below-defined compounds as effective inhibitors of human lung tryptase and, as such, are useful inthe treatment of asthma and acquired immunodeficiency syndrome (AIDS); said compounds being compounds of the formula

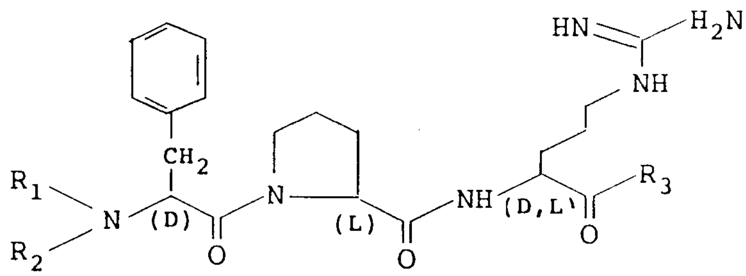

**XX**

the hydrates thereof, and the pharmaceutically acceptable salts thereof wherein

$R_1$ is H or $C_{1-7}$ alkyl,

$R_2$ is H or $C_{1-7}$ alkyl, or $R_1$ and $R_2$ taken together with the nitrogen atom to which they are attached form a 5- or 6-membered heterocycle,

$R_3$ is $CF_3$ or $CF_2CF_3$.

Using standard *in vitro* methodology for determining inhibitory activity of tryptase (Journal of Biological Chemistry, Vol. 261, June 5, pp. 7372-7379, 1986), the following results have been obtained.

| HUMAN LUNG TRYPTASE | | | |
|---|---|---|---|
| Rate constants[a] | $k_{on}$, $M^{-1}s^{-1}$ | $k_{off}$, $s^{-1}$ | $K_i$, M |
| D-Phe-Pro-Arg-$CF_3$ | 370 ± 40 | b | - |
| D-$CH_3$-Phe-Pro-Arg-$CF_3$ | 200 ± 10 | b | - |
| D-Phe-Pro-Arg-$CF_2CF_3$ | 1200 ± 40 | $6.8 \times 10^{-4} \pm 3.6 \times 10^{-5}$ | $6.0 \times 10^{-7}$ |

[a]Rate constants were determined by progress curve analysis using a non-linear regression program (ENZFITTER);
[b]In these cases, $k_{off}$ could not be determined accurately. Inhibition may be irreversible.

(It is to be noted that D-Phe-Pro-Arg-$CF_3$ and D-Phe-Pro-Arg-$CF_2CF_3$ are known compounds known to be useful as thrombin inhibitors. However, their use as tryptase inhibitors is novel and therefore their use in the treatment of asthma and AIDS is contemplated within this aspect of the invention).

Based upon the foregoing type assay, as well as by comparison with other compounds also known to possess tryptase inhibitory activity, it is to be found that the compounds of this invention, in their end-use application for the treatment of asthma will be about 1-100 mg per treatment; the dose regimen, of course, being dependent on the severity of the attack, the age and general condition of the patient, as well as other factors well appreciated by the attending diagnostician. In the treatment of AIDS, the dose range will be about 1-100 mg per kilogram of body weight per day, again allowing for the usual discretion of the attending diagnostician.

Although it may be possible that some of the administered tripeptides of this invention may survive passage through the gut following oral administration, it is preferred that the compounds be utilized via a non-oral administration. Subcutaneous, intravenous, intramuscular or intraperitoneal administrations, depot injections, implant preparations and such other non-oral methods are the preferred manners by which the compounds may be utilized. In those instances wherein asthma is being treated it is preferred to utilize metered dose aerosols or by application to the mucous membranes, (e.g. nose, throat and bronchial tubes) in an aerosol can containing a peptide derivative of this invention in the form of a spray or dry powder pharmaceutical formulation.

For a parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by Dow-Corning Corporation.

## Claims

**1.** A compound of the formula

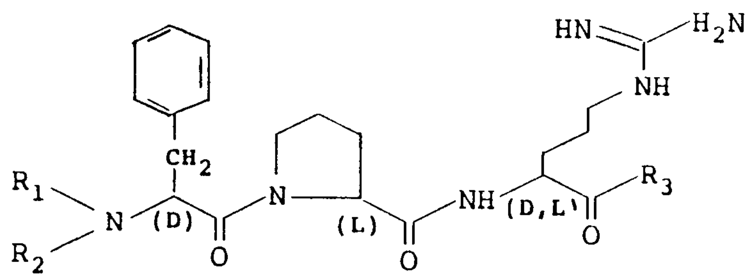

I

the hydrates thereof, and the pharmaceutically acceptable salts thereof wherein

$R_1$ is H or $C_{1-7}$ alkyl,

$R_2$ is H or $C_{1-7}$ alkyl, or $R_1$ and $R_2$ taken together with the nitrogen atom to which they are attached form a 5- or 6-membered heterocycle,

$R_3$ is $CF_3$ or $CF_2CF_3$, with the proviso that when $R_1$ and $R_2$ are both H, then $R_3$ is other than $CF_3$ or $CF_2CF_3$.

European Patent Office

# EUROPEAN SEARCH REPORT

Application Number

EP 91 40 0713

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 410 411 (MERRELL DOW)<br>* Page 8, line 37 - page 9, line 1 *<br>--- | 1 | C 07 K 5/08<br>A 61 K 37/64 |
| Y | CHEMICAL ABSTRACTS, vol. 110, 1989, page 709, abstract no. 76058k, Columbus, Ohio, US; H. ANGLIKER et al.: "Synthesis and properties of peptidyl derivatives of arginylfluoromethanes", & BIOCHEM. J. 1988, 256(2), 481-6<br>* Abstract *<br>--- | 1 | |
| Y | BIOCHEMISTRY, vol. 24, no. 8, 9th April 1985, pages 1813-1817, Washington, US; M.H. GELB et al.: "Fluoro ketone inhibitors of hydrolytic enzymes"<br>* Page 1814, column 1 *<br>--- | 1 | |
| Y | FEBS LETTERS, vol. 220, no. 2, August 1987, pages 299-301, Amsterdam, NL; H.L. sham et al.: "Highly potent and specific inhibitors of human renin"<br>* Pages 300-301: "Results and discussion" *<br>--- | 1 | TECHNICAL FIELDS SEARCHED (Int. Cl.5) |
| A | EP-A-0 185 390 (RICHTER GEDEON VEGYESZETI GYAR)<br>* Page 1, abstract *<br>----- | 1 | C 07 K<br>A 61 K |

The present search report has been drawn up for all claims

| Place of search | Date of completion of the search | Examiner |
|---|---|---|
| THE HAGUE | 07-11-1991 | KORSNER S.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)